# EUROPEAN PATENT APPLICATION

(11) **EP 2 926 803 A1**
(43) Date of publication of application: **07.10.2015**
(21) Application number: 14162764.6
(22) Date of filing: 31.03.2014
(51) Int. Cl.: A61K 8/60, A61Q 19/08, A61K 8/97

(54) **Use of crocin for reducing wrinkles**

(71) Applicant: Givaudan SA, 1214 Vernier (CH)
(72) Inventor: Lamy, Louis, 95100 Argenteuil (FR)
(74) Representative: McStea, John Anthony

(57) **Abstract**

An anti-wrinkling preparation comprising an effective wrinkle-reducing proportion of crocin.

## Description

This disclosure relates to a method skin care.

Wrinkles, folds in the skin, are a natural consequence of aging. They can be exacerbated by other factors, such as exposure to sun and smoking. While major wrinkling on the face is ultimately an unavoidable consequence of aging, it has long been desired to retard or eliminate the onset of wrinkling, such as so-called "crows' feet" around the corners of the eyes, which can make a person seem rather older than s/he actually is. To this end, there is a very substantial industry that provides all manner and compositions of anti-wrinkle preparations.

It has now been found that a particular preparation has particularly efficacious results against wrinkling. There is therefore provided an anti-wrinkling skin preparation comprising an effective wrinkle-reducing proportion of crocin. There is also provided a method of reducing the incidence of wrinkling in human skin, comprising the application to wrinkle-affected skin of a composition comprising an effective wrinkle-reducing proportion of crocin.

Crocin, the diester of the disaccharide gentiobiose and the dicarboxylic acid crocetin, is a carotenoid compound having the formula:

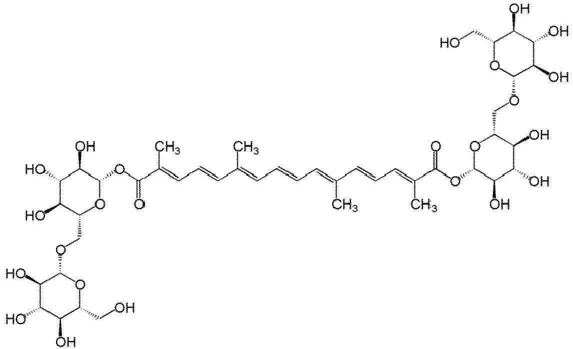

It occurs naturally in crocus and gardenia fruit. It is also responsible for the colour of saffron. It is known to have antioxidant properties, and studies have suggested anti-cancer properties. However, it is unexpected to find that it has an effect on wrinkles.

It is believed, without restricting the disclosure in any way, that crocin has the effect of stimulating the natural production of ceramides, waxy lipid molecules found in skin cell walls and whose presence is believed essential to smooth skin by its ability to retain water in the epidermis (ceramides are common ingredients in anti-wrinkling creams). It is postulated that crocin induces the activation of N-shingomyelanase, which in turn regulates ceramide production.

Crocin may be used as the pure compound. However, it is also possible, and particularly convenient, to use a natural extract of gardenia. Such extracts typically contain from 0,01 to 10 % by weight of crocin. It is also possible to provide extracts with an increased proportion of crocin, up to 80wt%. The provision of such an extract is well within the ordinary skill of the art.

The "effective wrinkle-reducing proportion" of crocin will depend on the extent of wrinkling to be treated, and it can vary between wide limits, but typically it will be from 0.001 % to 1 % by weight.

The crocin may be incorporated by any convenient method into any desirable anti-wrinkling preparation, which may be applied by any convenient means, for example by manual application, spraying or injection. In a typical example, it may form part of a cream to be applied topically to the skin, which cream may contain all the usual ingredients of skin creams in art-recognised proportions. They may include, but are not restricted to, glycerol, petrolatum, dimethicone, emulsifiers, preservatives, antioxidants, fragrances, and the like.

The application of such anti-wrinkling preparations have been found to reduce the severity of wrinkling, and in some cases eliminate it completely.

The disclosure is further described with reference to the following non-limiting examples, which depict particular embodiments.

The following cream was prepared:

| ingredients | Wt% |
|---|---|
| water | 73.7 |
| Glycerine | 5 |
| Propylene glycol | 0.5 |
| Butylene glycol | 0.3 |
| Polyacrylate crosspolymer-6 | 0.4 |
| EDTA | 0.1 |
| Liquid paraffin | 7 |
| Petrolatum oil | 2 |
| Stearyl alcohol | 1 |
| Glyceryl stearate | 0.8 |
| Gardenia Florida fruit extract/dipropylene glycol* | 4 |
| Fragrance | 0.3 |
| Phenoxyethanol/ethylhexylglycerin 90/10 | 0.9 |

| | |
|---|---|
| *0.1wt% in DPG. Extract contains 80wt% crocin. | |

The formulation, which contained 0.08 wt% crocin, was tested on 20 healthy female subjects having Caucasian skin types, all aged at least 45 and all having crows' feet fine lines. The duration of the test was 28 days from initial application.

The subjects were treated with both the abovementioned formulation and a placebo, which was identical to the formulation but containing no gardenia extract.The formulation was applied to one side of the face and the placebo to the other. The testing was conducted double-blind, with both applicators and the subjects being unaware of which was the formulation and which the placebo.

Measurement was by fast optical *in vivo* topometry, as described, for example, by Piche et al in Biomed Tech (Berl). 2000 Nov;45(11):317-22 and Rohr et al in SÖFW-Journal 124 (1998) 52-59. The technique provides a method which permits the gathering of three-dimensional information from the skin surface in an extremely short time (approximately 260 ms). The apparatus used was the DermaTOP system of Eotech S.A, using a Breuckmann 3D scanner and Eotech proprietary software.

The results were as follows:

**Table 1: In vivo fringe projection of the crows' feet (rugosity parameters)**

| | placebo | Cream with Gardenia |
|---|---|---|
| ST: maximum amplitude of the relief | 0% | -4% |
| SA: Average roughness | 1% | -7% |
| SQ: Roughness with respect to average quadratic variation | 1% | -7% |
| Stm; mean difference between peaks and valleys | 1% | -4% |

The measurements in Table 1 show the percentage variation of the area (in mm²) over the time of the test.

**Table 2: In vivo projection of the crows' feet wrinkles (morphology parameters)**

| | volume | area |
|---|---|---|
| placebo | 3% | 4% |
| Cream with Gardenia | -20% | -14% |
| Delta* | -23% | -18% |

| | | |
|---|---|---|
| * Difference between cream and placebo. | | |

The reduction in wrinkles brought about by the use of the cream containing the crocin can clearly be seen.

## Claims

1. An anti-wrinkling skin preparation comprising an effective wrinkle-reducing proportion of crocin.

2. A method of reducing the incidence of wrinkling in human skin, comprising the application to wrinkle-affected skin of a composition comprising an effective wrinkle-reducing proportion of crocin.
